# EUROPÄISCHE PATENTSCHRIFT

(11) **EP 2 504 300 B1**
(45) Veröffentlichungstag und Bekanntmachung des Hinweises auf die Patenterteilung: **15.01.2014**
(21) Anmeldenummer: 10782592.9
(22) Anmeldetag: 23.11.2010
(51) Int. Cl.: C07C 29/149, C07C 31/20

(54) **VERFAHREN ZUR HERSTELLUNG VON DIOLEN DURCH HYDRIERUNG EINES CARBONSÄURE ENTHALTENDEN GEMISCHES MITTELS KOBALT ENTHALTENDEN KATALYSATOREN**
METHOD FOR PRODUCING DIOLS BY HYDROGENATION OF A CARBOXYLIC ACID-CONTAINING MIXTURE USING COBALT-CONTAINING CATALYSTS
PROCÉDÉ DE PRÉPARATION DE DIOLS PAR HYDROGÉNATION D'UN MÉLANGE CONTENANT DE L'ACIDE CARBOXYLIQUE AU MOYEN DE CATALYSEURS AU COBALT

(30) Priorität: 26.11.2009 DE 102009047193
(43) Veröffentlichungstag der Anmeldung: 03.10.2012
(73) Patentinhaber: BASF SE, 67056 Ludwigshafen (DE)
(72) Erfinder: PINKOS, Rolf, 67098 Bad Dürkheim (DE); PAUL, Axel, 68623 Lampertheim (DE); FISCHER, Rolf-Hartmuth, 69121 Heidelberg (DE); DOSTALEK, Roman, 67271 Neuleiningen (DE)
(86) Internationale Anmeldenummer: PCT/EP2010/067973
(87) Internationale Veröffentlichungsnummer: WO 2011/064182

(56) Entgegenhaltungen:
- DE-A1- 2 321 101
- DE-A1- 19 756 171
- DE-B- 1 235 879

## Beschreibung

Die vorliegende Erfindung betrifft ein Verfahren zur Herstellung von Diolen durch Hydrierung eines Carbonsäure, Carbonsäureanhydride und/oder Carbonsäureester/Lactone enthaltendes Gemisch mit Hilfe eines Kobalt enthaltenden Katalysators, wobei dem Hydrierzulauf Alkali- und/oder Erdalkaliionen hinzugefügt werden und Alkali- und/oder Erdalkaliionen von Mineralsäuresalzen ausgeschlossen sind, und wobei die Säurezahl im Zulauf vor dem Katalysator, eingestellt durch das Verhältnis Zulauf zu Umlauf über den Reaktor oder die Reaktoren, zwischen 10 und 90 liegt.

In US 4,940,805, EP-A 304 696, EP-A 382 050, DE-AS 12 35 879 und DE-A 23 21 101 werden Kobalt und gegebenenfalls Mangan enthaltende Katalysatoren zur Hydrierung von Carbonsäuren, Carbonsäureanhydriden oder Carbonsäureestern/Lactonen zu den entsprechenden Diolen beschrieben. Nachteilig bei diesen Katalysatoren ist, dass diese in Gegenwart von Säuren und Wasser nicht stabil sind, sondern die Aktivkomponenten wie Co und gegebenenfalls Mn nach und nach verlieren. Dies wirkt sich negativ auf die Katalysatorstandzeit aus. Ferner kommt es an den sauren Katalysatorzentren zur Dehydratisierung und/oder Veretherung des während der Hydrierung entstehenden Diols und damit zu einem Ausbeuteverringerung des herzustellenden Diols. Durch die Bildung dieser Nebenprodukte muss das erhaltende Diol weiteren Reinigungsschritten wie Destillationen zugeführt werden, was wiederum die Ausbeute des erhaltenen Diols verringert.

In DE-A 12 35 879 ist ebenfalls die Hydrierung von Carbonsäuren an Kobalt und Mangan enthaltenden Katalysatoren beschrieben, wobei zur Verbesserung der Aktivität und Standzeit Säuren, die Polysäuren bilden können bzw. deren Alkali-, Erdalkali- oder Erdmetallsalze zugesetzt werden. Der Zusatz kann während der Katalysatorpräparation oder über den Zulauf, wie es in Beispiel 16 von DE-A 12 35 879 offenbart ist, erfolgen. In Beispiel 16 von DE-A 12 35 879 wird beschrieben, dass die Hydrierung eines Carbonsäuregemisches, das unter anderem Adipinsäure, Glutarsäure, Bernsteinsäure, 6-Hydroxycapronsäure, bereits hydriertes Carbonsäuregemisch sowie eine nicht näher definierte Mengen an rohen Monoalkoholen, deren Wassergehalt auf 7 % eingestellt wurde, enthält, mit 0,1 Gew.-% Na₃PO₄ umgesetzt wurde. Nachteilig bei diesem Verfahren ist, dass der Zusatz von Natriumphosphat als einem Alkalisalz einer Mineralsäure zum Hydrierzulauf keine Erhöhung der Aktivität und Standzeit des Katalysators bewirkt, sondern im Gegenteil zu verringerten Ausbeuten des gewünschten Endproduktes bei vermehrten Abbau der aktiven Katalysatormetalle führt.

Auch DE19756171 betrifft ein Verfahren zur Erhöhung der Katalysatoraktivität und die Vermeidung von Nebenreaktionen bei der Hydrierung von Carbonsäuren oder deren Derivaten durch Zudosierung von bestimmten basisch wirkenden Alkali- oder Erdalkaliverbindungen zum Hydrierfeed.

Die Aufgabe der vorliegenden Erfindung ist es daher, ein Verfahren zur Herstellung von Diolen bereit zu stellen, das es ermöglicht, den Austrag an Co- und gegebenenfalls Mn-Ionen aus dem eingesetzten Kobalt enthaltenden Katalysator zu verringern, somit die anfängliche Katalysatoraktivität möglichst lange zu erhalten und die Katalysatorstandzeit zu verlängern sowie die Weiterreaktion der gewünschten Diole zu Nebenprodukten, insbesondere zu Hexanol und, sofern wässrige Waschextrakte aus der Cyclohexanoxidation mit Luft verwendet werden, Cyclohexyl-1,6-hexandiolether, zu vermindern. Diese Eliminierungs- und Veretherungsreaktionen, die als Nebenreaktionen im Allgemeinen sauer katalysierte Reaktionen sind, gehen meist mit der Abspaltung von Wasser einher und bewirken eine Verringerung der Ausbeute des herzustellenden Diols. Eine weitere Aufgabe des erfindungsgemäßen Verfahrens ist somit auch die Ausbeute an dem gewünschten Diol, insbesondere 1,6-Hexandiol, zu erhöhen.

Diese Aufgabe wird gelöst durch ein Verfahren zur Herstellung von Diolen durch Hydrierung eines Carbonsäure, Carbonsäureanhydride und/oder Carbonsäureestern/Lactone enthaltenden Gemisches mittels eines Kobalt enthaltenden Katalysators, wobei dem Hydrierzulauf Alkali- und/oder Erdalkaliionen hinzugefügt werden und Alkali- und/oder Erdalkaliionen von Mineralsäuresalzen ausgeschlossen sind, und wobei die Säurezahl im Zulauf vor dem Katalysator, eingestellt durch das Verhältnis Zulauf zu Umlauf über den Reaktor oder die Reaktoren, zwischen 10 und 90 liegt.

Bei dem erfindungsgemäßen Verfahren wird ein Carbonsäure, Carbonsäureanhydrid und/oder Carbonsäureester/Lactone enthaltendes Gemisch mittels eines Kobalt enthaltenden Katalysators hydriert. Die in dem zu hydrierendem Gemisch enthaltenen Carbonsäure, Carbonsäureanhydrid und/oder Lactone können dabei ausgewählt sein aus der Gruppe von Maleinsäureanhydrid, Maleinsäure, Bernsteinsäureanhydrid, Bernsteinsäure, Glutarsäure, Zitronensäure, Itakonsäure, Adipinsäure, Hydroxycapronsäure, Caprolacton oder sind das wässrige Extrakt, das durch Sauerstoffoxidation von Cyclohexan zu Cyclohexanol/Cyclohexanon-Gemischen und anschließender Extraktion mit Wasser entsteht. Die Hydrierung kann in Substanz, d.h. z.B. in Schmelze, oder in Lösungsmitteln wie z.B. Alkoholen wie Methanol, Ethanol usw. oder inerten Lösungsmitteln wie Tetrahydrofuran, Dioxan oder Diethylether oder in Wasser durchgeführt werden. Im zu hydrierenden Gemisch können auch nicht cyclische Ester enthalten sein, allerdings nicht als Hauptkomponente. Bezogen auf die Säuren oder Anhydride oder Lactonen liegt deren Gehalt im Zulauf unter 20 Mol-%. Während der Hydrierung kann der Estergehalt ansteigen.

Wird nun dem Hydrierzulauf Alkali- und/oder Erdalkaliionen, die keine Salze einer Mineralsäure sind, hinzugefügt, so kann einerseits der Co- und gegebenenfalls Mn-Austrag vermindert und damit die Katalysatorstandzeit verlängert, andererseits die Aktivität des Katalysators und Selektivität der Hydrierreaktion nicht nur erhalten, sondern diese sogar gesteigert werden. Dies überrascht umso mehr, als die Menge des Zusatzes bei Weitem nicht ausreicht, um die in der Hydrierung anwesenden Säuren zu neutralisieren und damit die Nebenreaktionen zu vermindern.

Die Zugabe der Alkali- oder Erdalkaliionen kann in Form ihrer Verbindungen erfolgen, wobei Mineralsäuresalze, auch basisch wirkende, der Alkali- und Erdalkalimetalle jedoch ausgeschlossen sind. Als Alkaliionen sind dabei bevorzugt solche, die ausgewählt sind aus der Gruppe von Lithium, Natrium, Kalium, Rubidium und Cäsium, besonders bevorzugt sind Lithium, Natrium und Kalium, ganz besonders bevorzugt sind Natrium und Kalium. Als Erdalkaliionen sind dabei bevorzugt solche, die ausgewählt sind aus der Gruppe von Magnesium, Calcium, Strontium und Barium, besonders bevorzugt sind Magnesium und Calcium. Diese Alkali und/oder Erdalkalisalze werden bevorzugt in Form ihrer Oxide, Hydroxide, Alkoholate und Carboxylate zugegeben, wobei als Carboxylate die Carboxylate bevorzugt sind, die Carboxylate der Carbonsäuren der entsprechend zu hydrierenden Ester, Anhydride oder Carbonsäuren sind. Besonders bevorzugt werden die Alkali- und/oder Erdalkalisalze in Form ihrer Hydroxide und Carbonate zugegeben. Ganz besonders bevorzugt ist die Zugabe von Alkaliionen zum Hydrierzufluss.

Die erfindungsgemäße Zugabe der Alkali- und/oder Erdalkaliionen erfolgt in Mengen von, bezogen auf die Menge des Hydrierzulaufs, 10 bis 2000 ppm, bevorzugt 50 bis 1500 ppm, besonders bevorzugt 100 bis 800 ppm, ganz besonders bevorzugt sind 200 - 500 ppm.

Der Kobalt-Gehalt der Hydrierkatalysatoren, bezogen auf den Gesamtgewichtsgehalt des Katalysators, kann je nach Katalysatorart variieren und liegt im Bereich von 0,1 bis 99 Gew.-%. Der Kobalt-Gehalt der Hydrierkatalysatoren beträgt im Falle von Trägerkatalysatoren 0,1 bis 20 Gew.-%, vorzugsweise 0,5 bis 10 Gew.-%, im Falle von Vollkatalysatoren 10 bis 99 Gew.-%, vorzugsweise 20 bis 99 Gew.-%. Ist Mangan enthalten, so beträgt der Mangangehalt im Falle von Trägerkatalysatoren 0,1 bis 10 Gew.-%, vorzugsweise 0,2 bis 5 Gew.-%, im Falle von Vollkatalysatoren 0,5 bis 30 Gew-%, vorzugsweise 1 bis 20 Gew.-%. Neben den genannten Komponenten können auch noch andere Elemente anwesend sein, entweder in metallischer Form, oder als Oxide oder dergleichen. Metalle können auch in Form von Legierungen vorhanden sein. Genannt seinen beispielsweise Re, Cu, Fe, Mo, Ni und P. Des Weiteren sind Elemente möglich, die aufgrund des Herstellprozesses der vorgenannten Elemente aus Verunreinigungen im Katalysator auftreten oder zugesetzt werden. Im Allgemeinen liegt dann deren Gehalt unter 100 ppm. Ein bevorzugter Katalysator für das erfindungsgemäße Verfahren weist die Elemente Co, Cu, Mn, Mo, Na und P in Form ihrer Oxide in Anteilen von 60 - 70 Gew.-% Co, 15 - 25 Gew.-% Cu, 5 - 10 Gew.-% Mn, 2 - 5 Gew.-% Mo, 0,05 - 0,5 Gew.-% Na und 1 - 3 Gew.-% P auf.

Die Katalysatoren werden vor der eigentlichen Hydrierung aktiviert. D.h. die oxidischen Metallkomponenten werden zumindest zum Teil in die metallische Form überführt. Dies gilt v.a. für Co. Diese Aktivierung kann im Reaktor selbst erfolgen, jedoch auch an anderer Stelle, wobei die aktivierten Katalysatoren dann bevorzugt unter Lösemittel wie z.B. Wasser oder Pentandiol transportiert und eingebaut werden oder mittels Luft oberflächig passiviert in den Reaktor eingebaut werden. Bevorzugt werden die Co-Katalysatoren bei Temperaturen bis zu 350 °C im Wasserstoffstrom, dem ggf. Stickstoff beigemischt wird, aktiviert. Besonders bevorzugt sind Temperaturen bis 300°C.

Die Zugabe von Alkali- und/oder Erdalkalimetallionen nach dem erfindungsgemäßen Verfahren kann in bestimmten Abständen, bevorzugt aber kontinuierlich, erfolgen. Kontinuierlich ist bevorzugt, da der Zusatz an Alkali- und/oder Erdalkaliionen, selbst wenn sie sich bereits herstellungsbedingt auf oder im Katalysator befinden, durch den Hydrierungsprozess herausgewaschen werden. Werden dagegen die Alkali- und/oder Erdalkaliionen dem erfindungsgemäßen Verfahren von Beginn an kontinuierlich hinzugegeben, bevorzugt in Mengen von 10 bis 2000 ppm, so bleibt der Co- und gegebenenfalls Mn-Austrag auf niedrigem Niveau, und die Katalysatoraktivität fällt weniger schnell ab als ohne Zusatz. Werden Alkali- und/oder Erdalkaliionen zu einem bereits in Betrieb befindlichen Katalysator gegeben, so stellt man fest, dass die Co- und gegebenenfalls Mn-Austräge sinken, die Aktivität und Selektivität dagegen steigen.

Außerdem kann durch das erfindungsgemäße Verfahren die Sicherheit während der Hydrierung erhöht werden. Co-Katalysatoren zeigen unter Umständen die Tendenz zu unkontrollierten exothermen Reaktionen. Diese können durch die kontinuierliche Zugabe von Alkali- oder Erdalkaliionen unterdrückt werden.

Die Hydrierbedingungen, die ohne den erfindungsgemäßen Alkali- oder Erdalkalizusatz eingestellt worden sind, können auch mit dem Alkali- oder Erdalkalizusatz eingehalten werden. Ein Verfahren zur Hydrierung eines Carbonsäure, Carbonsäureanhydrid und/oder Lactone enthaltendes Gemisch mittels eines Kobalt enthaltenden Katalysators ohne dass weitere Alkali- und/oder Erdalkaliionen eingesetzt werden, kann daher ohne größere Probleme auf ein erfindungsgemäßes Verfahren umgestellt werden. Durch die Aktivitätserhöhung bei Zugabe von Alkali- und/oder Erdalkaliionen nach dem erfindungsgemäßen Verfahren kann der Zulauf und damit auch die Katalysatorbelastung gesteigert oder auch die erforderliche Hydriertemperatur abgesenkt werden.

Typische Reaktionsbedingungen für das erfindungsgemäße Verfahren sind Drücke die im Bereich von 100 bis 300 bar, bevorzugt im Bereich von 150 bis 290 bar liegen und Reaktionstemperaturen im Bereich von 150 bis 300°, bevorzugt im Bereich von 180 bis 250°C und Katalysatorbelastungen im Bereich von 0,05 bis 0,9 kg, besonders bevorzugt im Bereich von 0,1 bis 0,5 kg zu hydrierender Substanz/l Kat. x Stunde. Besonders bevorzugte Katalysatorbelastungen liegen bei 0,1 bis 0,4.

Dabei wird bei einer technischen Anlage in der Regel so verfahren, dass der Zulauf zusammen mit dem äußeren Umlauf, der zur Wärmezufuhr, meist zur Wärmeabfuhr der Reaktionswärme der Hydrierung dient, vor Erreichen der Katalysatorzone zusammengeführt und ggf. vermischt wird. Dabei sollte eine Mischtemperatur erreicht werden, die hoch genug ist, dass die Hydrierung ausreichend schnell erfolgt. Dies sind in der Regel Temperaturen zwischen 150 und 295°C, bevorzugt 180 bis 220°C. Entlang des Katalysatorbettes steigt dann durch die Reaktionswärme die Temperatur an. Im Regelfall sind dies 5 bis 70°C bevorzugt 5 bis 30°C. Dies hängt von dem Verhältnis von Zulauf zu Umlauf, der Reaktionswärme und des Umsatzes ab. Das Mengenverhältnis von Umlauf zu Zulauf liegt im Regelfall bei 1 : 1 bis 30 : 1, bevorzugt bei 5 : 1 bis 15 : 1. Die Reaktion kann auf mehrere Reaktoren aufgeteilt werden, die dabei parallel oder seriell in Riesel- und/oder Sumpffahrweise betrieben werden können. Dabei ist es vorteilhaft, den Hauptteil der Hydrierreaktion, über 50 %, bevorzugt über 80 %, besonders bevorzugt über 90 % in einem oder mehreren Reaktoren vorzunehmen, der oder die mit Umlauf betrieben werden. Der Rest der Hydrierreaktion, aber ohne Produktrückführung, ist vorteilhaft im geraden Durchgang in Sumpf- oder Rieselfahrweise durchzuführen.

Erfindungsgemäß liegt die Säurezahl vor Beginn der Hydrierung, d.h. direkt vor dem Katalysatorbett zwischen 10 und 90, bevorzugt zwischen 15 und 70, besonders bevorzugt zwischen 20 und 60. Diese Säurezahl wird eingestellt durch das Verhältnis Zulauf zu Umlauf über den Reaktor oder die Reaktoren. Wird beispielsweise eine Umlaufmenge von 50 to/h gefahren, mit einer Säurezahl von 5 und eine Zulaufmenge von 10 to/h mit einer Säurezahl von 300, so liegt die Säurezahl vor Erreichen des Katalysators bei ca. 51.

Die Säurezahl ist definiert als die Masse an Kaliumhydroxid in mg, die zur Neutralisation von 1 g einer Probe erforderlich ist (DIN 53402).

Liegt die Säurezahl im Bereich oberhalb von 90, so kommt es trotz Zudosieren von Alkali zu einem relativ raschen Nachlassen der Hydrieraktivität des Katalysators.

Liegt die Säurezahl unter 10, so bewirkt das Zudosieren von Alkali unter Umständen unerwünschte Ablagerung entweder auf dem Katalysator oder bei der Aufarbeitung des Hexandiols.

Es ist auch möglich, den Hauptteil der Reaktion nicht mit fest angeordnetem Katalysator, sondern in Suspensionsfahrweise vorzunehmen.

Der für die Hydrierung notwendige Wasserstoff wird vorteilhaft überstöchiometrisch zugeführt, wobei beispielsweise pro Säurefunktion mindestens zwei Wasserstoffe eingesetzt werden. Vorteilhaft liegt der Überschuss bei 1 bis 100 % Wasserstoff, bevorzugt sind 2 bis 20 %.

Der überschüssige Wasserstoff wird üblicherweise, ggf. zusammen mit anderen gasförmigen Produkten wie z.B. Methan, aus dem Reaktionssystem als Abgas ausgeschleust, es kann aber auch ohne Abgas gefahren werden, dann dient als Ausschleusung das unter den Reaktionsbedingungen gelöste Gas, das nach Entspannen und gegebenenfalls Abkühlen als sogenanntes Sprudelgas ausgeschleust wird.

Zwar wird im Allgemeinen versucht, den Überschuss an frisch zugeführten Wasserstoff so gering wie möglich zu halten, innerhalb des Reaktionssystems ist es jedoch bevorzugt, einen deutlichen Überschuss an Wasserstoff vorliegen zu haben. Dies kann erreicht werden beispielsweise durch Kreisgas, bei dem der gasförmige Reaktoraustrag ganz oder zum Teil über einen Kreisgasverdichter zurückgeführt wird. Bevorzugt ist die Reaktion, zumindest die Hauptreaktion in Rieselfahrweise durchzuführen, so dass möglichst viel Wasserstoff quasi stationär im System vorliegt, damit sich verbrauchter Wasserstoff möglichst schnell nachlösen kann. Sofern mit Kreisgas gefahren wird, kann dieser Kreisgasstrom im Verbund mit anderen Reaktionen, bei denen ebenfalls Wasserstoff benötigt wird, betrieben werden, z.B. die Hydrierung von 1,4-Butindiol zu 1,4-Butandiol. Dabei sollte ein gleiches Druckniveau verwendet werden.

Das Abgas aus der Hydrierung, gegebenenfalls auch das Sprudelgas, kann anderen Nutzern von Wasserstoff zur Verfügung gestellt werden, aber auch verbrannt werden, gegebenenfalls unter Energieerzeugung.

Die Reaktionsausträge der Hydrierung werden in der Regel mehrstufig destillativ bzw. rektikativ aufgearbeitet. Dies heißt unter Verwendung mehrere Kolonnen, bei denen Leichtsieder wie Wasser und niedersiedende Alkohole vom Produkt abgetrennt werden. Unter Leichtsiedern und niedrigsiedenden Alkoholen sind Produkte zu verstehen, die bei einem Druck von 100. bis 500 mbar einen Siedepunkte unterhalb von 100 °C haben. Danach wird das Produkt reindestilliert.

Das erfindungsgemäße Verfahren wird in den folgenden Beispielen näher erläutert. Die Angaben über die Metallgehalte wurden durch Atomabsorption bestimmt. Die Säure-und Esterzahlen sind titrimetrisch bestimmt (mg KOH/g Substanz).

Die in den Beispielen nachfolgend gemachten %-Angaben beziehen sich jeweils, sofern nichts anderes angegeben wird, auf das Gewicht.

### Beispiel 1

150 g/h eines Gemisches aus Adipinsäure (17 %), 6-Hydroxycapronsäure (16 %), Glutarsäure (2 %), 1,5 % 5-Hydroxypentansäure, 1 % Ameisensäure, 1 % 1,4-Cyclohexandiole, 1 % 1,2-Cyclohexandiole, 0,3 % Cyclohexanol/Cyclohexanon und anderen, mengenmäßig untergeordneten Verbindungen, wie es z.B. durch Sauerstoffoxidation von Cyclohexan zu Cyclohexanol/Cyclohexanon-Gemischen und anschließende Wasserwäsche erhalten werden kann (ca. 45 % Wasser), werden bei 230 °C/250 bar in einem 300 ml Rohrreaktor, in dem sich 200 ml Katalysator (66% CoO, 20% CuO, 7,3% Mn₃O₄, 3,6% MoO₃, 0,1% Na₂O, 3% H₃PO₄, Herstellung nach DE-A 23 21 101; 4 mm Stränge; Aktivierung mit Wasserstoff bis 300 °C) befinden, in Rieselfahrweise hydriert. Der Reaktoraustrag wird in einem Abscheider von überschüssigem Wasserstoff abgetrennt (Abgasmenge 100 l/h) und gelangt einerseits über eine Pumpe als Kreislaufstrom wieder auf den Kopf des Reaktors, wo er mit dem Zulaufstrom vereinigt wird (Zulauf: Umlauf = 1 : 10), andererseits in ein Austragsgefäß. Die Säurezahl vor dem Katalysator lag im Bereich von 70 - 75. Die Austräge wurden tageweise gaschromatographisch (Gew.-%, Methode mit innerem Standard) analysiert. Nach 30 Tagen Versuchszeit wurden folgende Werte im Austrag festgestellt: 1,6-Hexandiol: 15,7%, 6-Hydroxycapronsäure: 7,4%, 1,5-Pentandiol: 3,3% Hexanol: 1,8%, Cyclohexyl-1,6-hexandiolether: 0,1 %. Die Kobalt- und Mangangehalte im Austrag betrugen je ca. 20 ppm. Natrium war bereits nach 5 Versuchstagen unter 3 ppm (Nachweisgrenze) abgesunken. Die destillative Reinigung des Austrags ergab eine 1,6-Hexandiolreinheit von 97,5 % neben 2 % 1,4-Cyclohexandiolen, 0,2 % 1,5-Pentandiol, 0,1 % 1,2-Cyclohexandiolen, 0,08 % Cyclohexl-1-,6-hexandiolether, sowie weitere, unter 500 ppm liegende Verbindungen.

Danach wurden dem Zulauf NaOH zugemischt, so dass der NaOH-Gehalt bei 1000 ppm lag. Das erhaltene Gemisch wurde hydriert (Säurezahlen vor dem Katalysator zwischen 60 und 65). In den folgenden Tagen stiegen die Ausbeuten der Wertprodukte (z. B. 1,6-Hexandiol) an, während sich die Bildung des Nebenproduktes Hexanol verringerte. Nach 35 Tagen Versuchszeit, d. h. nach 5 Tagen NaOH-Zulauf, wurde folgende Analyse des Austrags erhalten: 1,6-Hexandiol: 18,5%, 6-Hydroxycapronsäure: 6,7%, 1,5-Pentandiol: 4%, Hexanol: 1,4%. Der Cyclohexy-1,6-Hexandiolether konnte im Rohgemisch aufgrund sehr geringer Mengen nicht sicher nachgewiesen werden. Die Kobalt- und Mangangehalte im Austrag betrugen 5 bzw. 11 ppm. Die destillative Aufarbeitung ergab ein ähnliches Bild wie ohne NaOH-Dosierung, allerdings lag der Gehalt an Cyclohexyl-1,6-Hexandiolether unter 100 ppm.

### Beispiel 2

In einem 2,5 l Rohrreaktor wurde an 2,4 l des gleichen Katalysators wie in Beispiel 1 die dort beschriebene Carbonsäurelösung hydriert. Allerdings wurde der Na-Gehalt des Austrages durch NaOH-Zugabe zur Zulauflösung von Beginn an auf 400 - 500 ppm eingestellt. Die Reaktoreingangstemperatur betrug 230 °C, der Abgasstrom betrug 250 l/h. Nach 5 Tagen Versuchszeit erhielt man, bei einem Zulauf von 1133 g/h, folgende Produktzusammensetzung: 28% Hexandiol, 1,4% 6-Hydroxycapronsäure und 1,4% Hexanol. Der Co-Austrag war unter 1 ppm, Mn 85 ppm.

In den folgenden Tagen wurde ein Zulauf von 1400 g/h eingestellt (Säurezahlen vor dem Katalysator ca. 55). Die Hexandiolgehalte im Austrag lagen dabei zwischen 27 und 28%. Der Hydroxycapronsäuregehalt lag bei ca. 2%, Hexanol 1,3%.

Am 17. Versuchstag wurden 27% Hexandiol, 2,3% Hydroxycapronsäure und 1,3% Hexanol, unter 1 ppm Co, 37 ppm Mn und 430 ppm Na im Austrag gefunden. Danach wurde die Natriumzufuhr gestoppt. Bereits 24 h später (18. Versuchstag) sank der Na-Gehalt im Austrag auf 7 ppm, dagegen stieg der Co-Austrag auf 1 ppm, Mn auf 75 ppm. Gleichzeitig fiel der Hexandiolgehalt auf 26%, die Hydroxycapronsäure blieb bei 2,3%, Hexanol stieg auf 1,4%. Diese Bedingungen wurden bis zum 24. Versuchstag beibehalten. Dabei sank der Na-Gehalt auf 4 ppm, der Co-Austrag stieg auf 2 ppm, Mn fiel auf 50 ppm. Der Hexandiolgehalt fiel kontinuierlich bis auf 24,8%, die Hydroxycapronsäure stieg auf 3%, Hexanol auf 1,5.

Am 25. Versuchstag wurden wieder Na-Ionen zudosiert, diesmal jedoch nicht erfindungsgemäß in Form von NaOH, sondern als Na₃PO₄.

Bereits nach 24 h war der Hexandiolgehalt auf 23,4% gefallen, die Hydroxycapronsäure bei 3,6%, Hexanol bei 1,6%. Der Co-Austrag betrug 2 ppm, Mn 4 ppm, Na 480 ppm. Nach weiteren 28 h war der Hexandiolgehalt bereits auf 18,8% gefallen. Hydroxycapronsäure 5%, Hexanol 2,2%. Außerdem war der Reaktoraustrag zweiphasig. Daraufhin wurde der Versuch abgebrochen.

Es zeigte sich somit, dass das Natriumsalz der Mineralsäure H₃PO₄ keine erfindungsgemäße Fähigkeit aufweist.

### Beispiel 3 zum Vergleich

Beispiel 2 (Anfangseinstellung) wurde wiederholt, mit dem Unterschied, dass das Zulauf- zu Umlaufverhältnis 1 zu 6 und die Säurezahl vor dem Katalysator nach 5 Tagen Versuchszeit bei ca. 95 lag. Dies Säurezahl stieg in der Folgezeit stetig an und es fanden sich nach 30 Versuchstagen ca. 5 ppm Co und ca. 100 ppm Mn im Austrag. Kurze Zeit danach wurde der Versuch abgebrochen, da der Austrag wieder zweiphasig war und der Hexandiolgehalt im Austrag nur noch 15 % betrug.

## Patentansprüche

1. Verfahren zur Herstellung von Diolen durch Hydrierung eines Carbonsäure, Carbonsäureanhydride und/oder Lactone enthaltenden Gemisches mittels eines Kobalt enthaltenden Katalysators, wobei dem Hydrierzulauf Alkali- und/oder Erdalkaliionen hinzugefügt werden und Alkali- und/oder Erdalkaliionen von Mineralsäuresalzen ausgeschlossen sind, und wobei die Säurezahl im Zulauf vor dem Katalysator, eingestellt durch das Verhältnis Zulauf zu Umlauf über den Reaktor oder die Reaktoren, zwischen 10 und 90 liegt.

2. Verfahren nach Anspruch 1, wobei das Carbonsäure, Carbonsäureanhydrid und/oder Lactone enthaltende Gemisch Adipinsäure enthält und die Hydrierung zur Herstellung von 1,6-Hexandiol, genutzt wird.

3. Verfahren nach einem der Ansprüche 1 bis 2, wobei die Mengen an Alkali-und/oder Erdalkaliionen, die dem Hydrierzulauf zugefügt werden, im Bereich von 10 bis 2000 ppm, bezogen auf den Hydrierzulauf, beträgt.

4. Verfahren nach einem der Ansprüche 1 bis 3, wobei die hinzugefügten Alkali-und/oder Erdalkaliionen ausgewählt sind aus der Gruppe von Lithium, Natrium, Kalium, Rubidium, Cäsium, Magnesium, Calcium, Strontium und Barium.

5. Verfahren nach einem der Ansprüche 1 bis 4, wobei die hinzugefügten Alkali-und/oder Erdalkaliionen dem Hydrierzulauf in Form ihrer Oxide, Hydroxide und Carboxylate ausgewählt sind.

6. Verfahren nach einem der Ansprüche 1 bis 5, wobei der Kobalt enthaltende Katalysator einen Kobaltgehalt im Bereich von 0,1 bis 99 Gew.-% bezogen auf das Gesamtgewicht es Katalysators enthält.

7. Verfahren nach einem der Ansprüche 1 bis 6, wobei der Kobalt enthaltende Katalysator zusätzlich noch Mangan enthält.

8. Verfahren nach einem der Ansprüche 1 bis 7, wobei die Hydrierung bei Drücken im Bereich von 100 bis 300 bar und Temperaturen im Bereich von 150 bis 300 °C durchgeführt wird.

9. Verfahren nach einem der Ansprüche 1 bis 8, wobei über 50 % der Hydrierreaktion in einem oder mehreren Reaktoren vorgenommen wird, der oder die mit Umlauf betrieben werden und der Rest der Hydrierreaktion im geraden Durchgang in Sumpf- oder Rieselfahrweise durchgeführt wird.

## Claims

1. A process for preparing diols by hydrogenating a mixture comprising carboxylic acid, carboxylic anhydrides and/or lactones by means of a cobalt-comprising catalyst, wherein alkali metal and/or alkaline earth metal ions are added to the hydrogenation feed, excluding alkali metal and/or alkaline earth metal ions of mineral acid salts, and wherein the acid number in the feed upstream of the catalyst, established by the ratio of feed to circulation over the reactor or the reactors, is between 10 and 90.

2. The process according to claim 1, wherein the mixture comprising carboxylic acid, carboxylic anhydride and/or lactones comprises adipic acid, and the hydrogenation is used to prepare 1,6-hexanediol.

3. The process according to either of claims 1 and 2, wherein the amounts of alkali metal and/or alkaline earth metal ions which are added to the hydrogenation feed are in the range from 10 to 2000 ppm, based on the hydrogenation feed.

4. The process according to any of claims 1 to 3, wherein the added alkali metal and/or alkaline earth metal ions are selected from the group of lithium, sodium, potassium, rubidium, cesium, magnesium, calcium, strontium and barium.

5. The process according to any of claims 1 to 4, wherein the added alkali metal and/or alkaline earth metal ions are added to the hydrogenation feed in the form of the oxides, hydroxides and carboxylates thereof.

6. The process according to any of claims 1 to 5, wherein the cobalt-comprising catalyst comprises a cobalt content in the range from 0.1 to 99% by weight, based on the total weight of the catalyst.

7. The process according to any of claims 1 to 6, wherein the cobalt-comprising catalyst additionally comprises manganese.

8. The process according to any of claims 1 to 7, wherein the hydrogenation is performed at pressures in the range from 100 to 300 bar and temperatures in the range from 150 to 300°C.

9. The process according to any of claims 1 to 8, wherein more than 50% of the hydrogenation reaction is undertaken in one or more reactors which is/are operated with circulation, and the rest of the hydrogenation reaction in straight pass in liquid phase mode or trickle mode.

## Revendications

1. Procédé pour la préparation de diols par hydrogénation d'un mélange contenant de l'acide carboxylique, des anhydrides d'acide carboxylique et/ou des lactones au moyen d'un catalyseur contenant du cobalt, des ions de métal alcalin et/ou de métal alcalino-terreux étant ajoutés à l'alimentation de l'hydrogénation et les ions de métal alcalin et/ou de métal alcalino-terreux de sels d'acides minéraux étant exclus et l'indice d'acide dans l'alimentation en amont du catalyseur, réglé par le rapport alimentation à circulation sur le ou les réacteurs, étant situé entre 10 et 90.

2. Procédé selon la revendication 1, le mélange contenant de l'acide carboxylique, de l'anhydride d'acide carboxylique et/ou des lactones contenant de l'acide adipique et l'hydrogénation étant utilisée pour la préparation de 1,6-hexanediol.

3. Procédé selon l'une quelconque des revendications 1 à 2, les quantités d'ions de métal alcalin et/ou de métal alcalino-terreux, qui sont ajoutées à l'alimentation de l'hydrogénation, étant situées dans la plage de 10 à 2000 ppm, par rapport à l'alimentation et l'hydrogénation.

4. Procédé selon l'une quelconque des revendications 1 à 3, les ions de métal alcalin et/ou de métal alcalino-terreux ajoutés étant choisis dans le groupe formé par le lithium, le sodium, le potassium, le rubidium, le césium, le magnésium, le calcium, le strontium et le baryum.

5. Procédé selon l'une quelconque des revendications 1 à 4, les ions de métal alcalin et/ou de métal alcalino-terreux étant ajoutés à l'alimentation de l'hydrogénation sous forme de leurs oxydes, hydroxydes et carboxylates.

6. Procédé selon l'une quelconque des revendications 1 à 5, le catalyseur contenant du cobalt présentant une teneur en cobalt dans la plage de 0,1 à 99% en poids par rapport au poids total du catalyseur.

7. Procédé selon l'une quelconque des revendications 1 à 6, le catalyseur contenant du cobalt contenant en outre encore du manganèse.

8. Procédé selon l'une quelconque des revendications 1 à 7, l'hydrogénation étant réalisée à des pressions dans la plage de 100 à 300 bars et des températures dans la plage de 150 à 300°C.

9. Procédé selon l'une quelconque des revendications 1 à 8, plus de 50% de la réaction d'hydrogénation étant réalisés dans un ou plusieurs réacteurs qui est/sont exploité(s) avec une circulation et le reste de la réaction d'hydrogénation étant réalisé en passage droit dans un mode opératoire avec un fond ou un ruissellement.
